# EUROPEAN PATENT APPLICATION

(11) **EP 1 767 530 A1**
(43) Date of publication of application: **28.03.2007**
(21) Application number: 05757756.1
(22) Date of filing: 05.07.2005
(51) Int. Cl.: C07D 263/56, A01N 43/76, A01N 47/02, C07B 61/00

(54) **BENZOXAZOLES**

(30) Priority: 07.07.2004 JP 2004201006
(71) Applicant: KYOYU AGRI CO., LTD, Kawasaki-shi Kanagawa 213-0002 (JP)
(72) Inventor: SUZUKI, Junji, Kyoyu Agri Co., Ltd., Nagano-shi, Nagano 381-0006 (JP); TAKAHASHI, Katsuhiro, Kyoyu Agri Co., Ltd., Nagano-shi, Nagano 381-0006 (JP); FUKUDA, S., Ube Res. Lab., Ube Industries, Ltd., Ube-shi, Yamaguchi 755-8633 (JP)
(74) Representative: Albrecht, Thomas
(86) International application number: PCT/JP2005/012718
(87) International publication number: WO 2006/004215

(57) **Abstract**

The invention discloses benzoxazole compounds which are represented by the following formula (1): 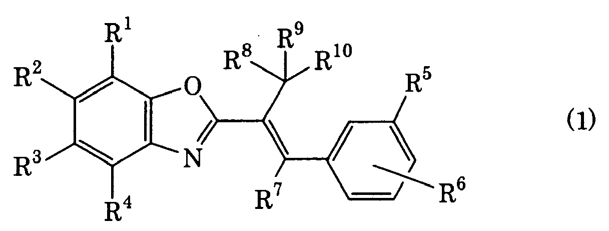 in which R¹-R⁴ stand for H, halogen, NO₂, CN, C₁₋₄ alkyl, C₁₋₄ haloalkyl, C₁₋₄ haloalkoxy, C₂₋₅ alkanoyl, formyl, C₂₋₆ alkoxycarbonyl, carboxyl or the like; R⁵ stands for C₁₋₄ haloalkyl, CN; R⁶ stands for H, halogen, CN, C₁₋₄ haloalkyl; R⁷ stands for H, halogen, C₁₋₄ alkyl; R⁸ and R⁹ each independently stands for H, C₁₋₄ alkyl; R¹⁰ stands for halogen, CN, R¹² X where R¹² stands for C₁₋₁₀ alkyl, C₁₋₆ haloalkyl, C₃₋₆ cycloalkyl, C₂₋₇ alkanoyl, formyl and X stands for O, S and which are useful as herbicide.

## Description

### Technical Field

This invention relates to benzoxazole compounds, methods for making the same and their use as herbicide.

### Background Art

It is known that certain kind of benzoxazole compounds are effective as herbicide (e.g., see JP 2002-155063A).

### Disclosure of the Invention

The main object of the present invention is to provide novel benzoxazole compounds which are useful as herbicide.

We have now discovered that the novel benzoxazole compounds represented by the following formula (1) are effective as herbicide, and completed the present invention.

Thus, the present invention provides benzoxazole compounds represented by the formula (1), 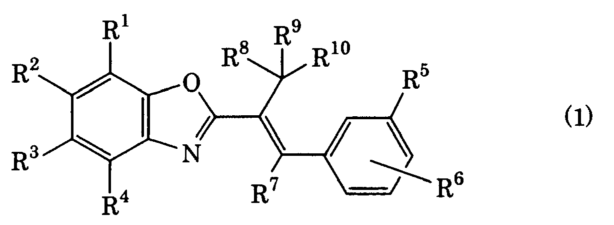 in the formula,
R¹ stands for hydrogen, halogen or C₁₋₄ alkyl,
R² stands for hydrogen, halogen, C₁₋₄ haloalkyl or C₁₋₄ alkyl,
R³ stands for hydrogen, C₁₋₄ haloalkyl, halogen, nitro, C₁₋₄ alkyl, cyano, R¹¹S(O)ₙ, C₁₋₄ haloalkoxy, C₂₋₅ alkanoyl, formyl, C₂₋₆ alkoxycarbonyl or carboxyl, where R¹¹ standing for C₁₋₄ alkyl and n being an integer of 0 - 2,
R⁴ stands for hydrogen, halogen or C₁₋₄ alkyl,
R⁵ stands for C₁₋₄ haloalkyl or cyano,
R⁶ stands for hydrogen, halogen, cyano or C₁₋₄ haloalkyl,
R⁷ stands for hydrogen, halogen or C₁₋₄ alkyl,
R⁸ and R⁹ each independently stands for hydrogen or C₁₋₄ alkyl,
R¹⁰ stands for halogen, cyano or R¹² X, here R¹² standing for C₁₋₁₀ alkyl, C₁₋₆ haloalkyl, C₃₋₆ cycloalkyl, C₂₋₇ alkanoyl, C₂₋₇ haloalkanoyl or formyl; and X standing for oxygen or sulfur.

The benzoxazole compounds of the present invention which are represented by the above formula (1) exhibit potent herbicidal effect and are useful as herbicide.

Hereinafter the invention is explained in further details.

In the following explanation of the present invention, those compounds identified by chemical formulae are indicated respectively by their chemical formula number, e.g., compounds of the above formula (1) are indicated as "compound (1)".

In the present specification,
"halogen" includes fluorine, chlorine, bromine and iodine atoms.
"Alkyl" can be of straight chain or branched chain, and as examples of which methyl, ethyl, n- or iso-propyl, n-, iso-, sec- or tert-butyl, n-pentyl, n-hexyl, n-octyl, 2-ethylhexyl, n-decyl and the like can be named.
"Haloalkyl" is a group in which at least one hydrogen atom of the alkyl group is substituted with halogen atom, examples of which including chloromethyl, dichloromethyl, trifluoromethyl, chloroethyl, dichloroethyl, trifluoroethyl, tetrafluoropropyl, bromoethyl, bromopropyl, chlorobutyl, chlorohexyl, perfluorohexyl and the like.
"Alkoxy" is an alkyl-O- group whose alkyl moiety has the above signification, examples of which including methoxy, ethoxy, n- or iso-propoxy, n-, iso-, sec- or tert-butoxy, pentyloxy, hexyloxy, heptyloxy, octyloxy, decyloxy and the like.
"Haloalkoxy" is a haloalkyl-O- group whose haloalkyl moiety has the above signification, examples of which including trifluoromethoxy, trifluoroethoxy, tetrafluoropropoxy, perfluorohexyloxy and the like.
"Alkoxycarbonyl" is an alkoxy-CO- group whose alkoxy moiety has the above signification, examples of which including methoxycarbonyl, ethoxycarbonyl, propoxycarbonyl, butoxycarbonyl, pentyloxycarbonyl and the like.
"Cycloalkyl" includes cyclopropyl, cyclopentyl, cyclohexyl and the like.
"Alkanoyl" is an alkyl-CO- group whose alkyl moiety has the above signification, examples of which including acetyl, propionyl, butyryl, iso-butyryl, valeryl, iso-valeryl, pivaloyl and the like.
"Haloalkanoyl" is an alkanoyl group whose at least one hydrogen atom is substituted with halogen, examples of which including chloroacetyl, trifluoroacetyl, chloropropionyl, bromopropionyl, chlorohexanoyl, chloroheptanoyl and the like.

In the formula (1), preferably
R¹ is hydrogen, halogen or C₁₋₃ alkyl,
R² is hydrogen, halogen, C₁₋₃ haloalkyl or C₁₋₃ alkyl,
R³ is hydrogen, C₁₋₃ haloalkyl, halogen, nitro, C₁₋₄ alkyl, cyano, R¹¹S(O)ₙ, C₁₋₃ haloalkoxy, C₂₋₃ alkanoyl, C₂₋₄ alkoxycarbonyl or carboxyl, wherein R¹¹ is C₁₋₃ alkyl and n is an integer of 0 - 2,
R⁴ is hydrogen, halogen or C₁₋₃ alkyl,
R⁵ is C₁₋₃ haloalkyl or cyano,
R⁶ is hydrogen, halogen, cyano or C₁₋₃ haloalkyl,
R⁷ is hydrogen, halogen or C₁₋₃ alkyl,
R⁸ and R⁹ are hydrogen or C₁₋₃ alkyl, independently of each other,
R¹⁰ is halogen, cyano or R¹² X, wherein R¹² is C₁₋₁₀ alkyl, C₁₋₆ haloalkyl, cyclohexyl, C₂₋₅ alkanoyl, C₂₋₅ haloalkanoyl or formyl, and X is oxygen or sulfur.

Still more preferably,
R¹ is hydrogen or halogen,
R² is hydrogen, halogen or C₁₋₃ haloalkyl,
R³ is halogen, cyano, C₁₋₃ haloalkyl, C₁₋₃ haloalkoxy, C₁₋₄ alkyl or C₂₋₃ alkanoyl,
R⁴ is hydrogen,
R⁵ is C₁₋₃ haloalkyl,
R⁶ is hydrogen or halogen,
R⁷ is hydrogen,
R⁸ and R⁹ each is hydrogen,

R¹⁰ is halogen or R¹²X, wherein R¹² is C₁₋₁₀ alkyl, C₁₋₆ haloalkyl, cyclohexyl or formyl, and X is oxygen or sulfur.

In particular, the most convenient combination of the substituents are as follows:
R¹ is hydrogen, fluorine or chlorine,
R² is hydrogen, fluorine, chlorine or trifluoromethyl,
R³ is fluorine, chlorine, bromine, iodine, trifluoromethyl, cyano, tert-butyl or acetyl,
R⁴ is hydrogen,
R⁵ is trifluoromethyl,
R⁶ is hydrogen, fluorine or chlorine,
R⁷ is hydrogen,
R⁸ and R⁹ each is hydrogen,
R¹⁰ is R¹² X, wherein R¹² is methyl, ethyl, n-propyl, iso-propyl, n-hexyl, n-octyl, n-decyl, 2,2,2-trifluoroethyl, 2,2,3,3-tetrafluoropropyl, 2-chloromethyl, 4-chloro-n-butyl, 6-chloro-n-hexyl or cyclohexyl, and X is oxygen.

Among compounds (1), the following are preferred in respect of their herbicidal activity:
(i) compounds of the formula (1) in which R¹, R², R⁴ and R⁶ - R⁹ each stands for hydrogen; R³ stands for halogen; R⁵ stands for C₁₋₄ haloalkyl; and R¹⁰ stands for C₁₋₁₀ alkoxy; for example, referring to the later appearing Table 1, Compounds 3, 11, 27, 29, 34, 39 and 41:
(ii) compounds of the formula (1) in which R¹, R², R⁴ and R⁷ - R⁹ each stands for hydrogen; R³ stands for halogen; R⁵ stands for C₁₋₄ haloalkyl; R⁶ stands for halogen; and R¹⁰ stands for C₁₋₁₀ alkoxy; for example, Compounds 8, 9, 26, 35, 40 and 42 in the later appearing Table 1:
(iii) compounds of the formula (1) in which R¹, R², R⁴ and R⁶ - R⁹ each stands for hydrogen, R³ stands for C₁₋₄ haloalkyl; R₅ stands for C₁₋₄ haloalkyl; and R¹⁰ stands for C₁₋₁₀ alkoxy; for example, Compounds 52, 54, 56, 60, 62 and 64 in the later appearing Table 1:
(iv) compounds of the formula (1) in which R¹, R², R⁴ and R⁶ - R⁹ each stands for hydrogen; R³ stands for C₁₋₄ haloalkyl, R⁵ stands for C₁₋₄ haloalkyl; and R¹⁰ stands for C₁₋₆ haloalkoxy; for example, Compounds 85, 87, 89, 91 and 93 in the later appearing Table 1:
(v) compounds of the formula (1) in which R², R⁴ and R⁶ - R⁹ each stands for hydrogen; R¹ and R³ each stands for halogen; R⁵ stands for C₁₋₄ haloalkyl; and R¹⁰ stands for C₁₋₆ alkoxy; for example, Compounds 106, 108, 109, 112, 113 and 114 in the later appearing Table 1:
(vi) compounds of the formula (1) in which R¹, R⁴ and R⁶ - R⁹ each stands for hydrogen; R² and R³ each stands for halogen; R⁵ stands for C₁₋₄ haloalkyl; and R¹⁰ stands for C₁₋₁₀ alkoxy; for example, Compounds 116, 119, 125, 127 and 133 in the later appearing Table 1:
(vii) compounds of the formula (1) in which R¹, R², R⁴ and R⁶ - R⁹ each stands for hydrogen; R³ stands for C₁₋₄ alkyl; R⁵ stands for C₁₋₄ haloalkyl; and R¹⁰ stands for C₁₋₁₀ alkoxy; for example, Compounds 151 and 153 in the later appearing Table 1:
(viii) compounds of the formula (1) in which R¹, R⁴ and R⁶ - R⁹ each stands for hydrogen; R² stands for halogen; R³ stands for cyano; R⁵ stands for C₁₋₄ haloalkyl; and R¹⁰ stands for C₁₋₁₀ alkoxy; for example, Compounds 117, 132 and 179 in the later appearing Table 1:
(ix) compounds of the formula (1) in which R¹, R⁴ and R⁶ - R⁹ each stands for hydrogen; R² stands for C₁₋₄ haloalkyl; R³ stands for halogen; R⁵ stands for C₁₋₄ haloalkyl; and R¹⁰ stands for C₁₋₁₀ alkoxy; for example, Compounds 139 and 141 in the later appearing Table 1.

Those compounds (1) provided by the present invention can be prepared, for example, by any of the hereinafter described methods (a), (b) or (c).

### Method (a):

A method for preparing the compounds (1), which comprises reacting a compound of the following formula (2): 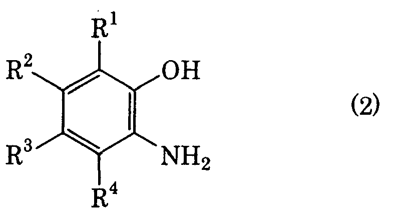 in which R¹-R⁴ have the same significations as given in the foregoing, with a compound represented by the following formula (3): 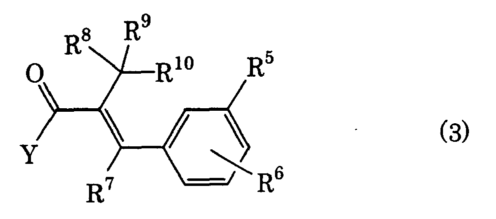 in which R⁵-R¹⁰ have the same significations as given in the foregoing; and Y stands for halogen, preferably chlorine or bromine; in the presence of a base or acid catalyst.

### Method (b):

A method for preparing the compounds (1) in which R¹⁰ stands for halogen, i.e., compounds of the following formula (5): 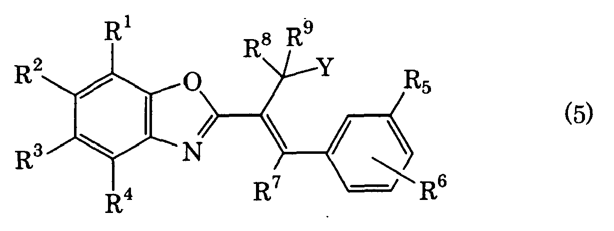 in which R¹-R⁹ have the earlier given definitions; and Y stands for halogen,
which comprises reacting a compound of the following formula (4): 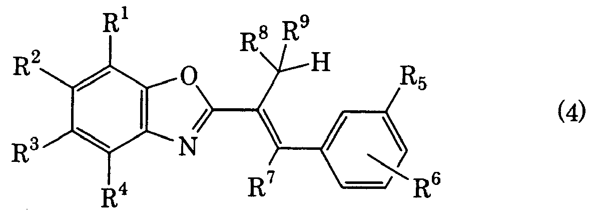 in which R¹-R⁹ have the earlier given definitions with a halogenating agent.

### Method (c):

A method for preparing the compounds (1) in which R¹⁰ stands for R¹²X, i.e., compounds of the following formula (7): 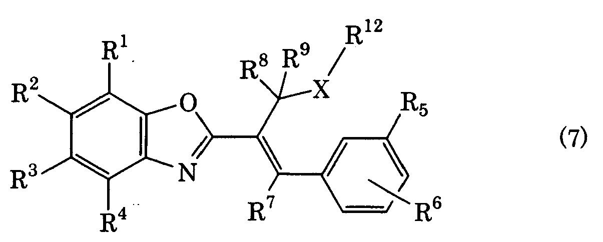 in which R¹-R⁹, R¹² and X have the earlier given definitions which comprises reacting the above compound (5) with a compound represented by the following formula (6):

R¹²-X-M (6)

in which R¹² and X have the earlier given definitions; and M stands for hydrogen or alkali metal, for example, potassium or sodium,
in the presence of a base catalyst.

Compounds (2) which are used as the starting material in the Method (a) are per se known compounds (see, for example, JP 2002-155063A), examples of which include: 2-amino-4- chlorophenol, 2-amino-4-trifluoromethylphenol, 2-amino-4,5- difluorophenol and the like.

Also compounds (3) which are used as the starting material are either per se known compounds or can be synthesized in the manner similar to the per se known compounds (see, for example, J. Org. Chem. 1995, Vol. 60, 4635). As compound (3), for example, (E)-2-(methoxymethyl)-(3-trifluoromethylphenyl)acrylic acid chloride, (E)-2-(ethoxymethyl)-(3-trifluoromethylphenyl)acryic acid bromide, (E)-2-(methoxymethyl)-(4-fluoro-3-trifluoromethylphenyl)acrylic acid chloride and the like can be named.

As the base catalyst useful in the occasion of reacting compound (2) with compound (3), for example, organic bases such as triethylamine, pyridine, 4-N,N-dimethylaminopyridine, N,N-dimethylaniline, 1,4-diazabicyclo[2.2.2]octane, 1,8-diazabicyclo-[5.4.0]undec-7-ene and the like; alkali metal alkoxides such as sodium methoxide, sodium ethoxide, potassium-t-butoxide and the like; inorganic bases such as sodium hydride, potassium hydride, sodium amide, sodium hydroxide, potassium hydroxide, potassium carbonate, sodium carbonate, potassium hydrogencarbonate, sodium hydrogencarbonate and the like; and organometal amides such as lithium diisopropylamide, bistrimethylsilyllithium amide and the like can be named. As the acid catalyst, for example, mineral acid such as hydrochloric acid, sulfuric acid, nitric acid and the like; organic acids such as formic acid, acetic acid, propionic acid, methanesulfonic acid, benzenesulfonic acid, p-toluenesulfonic acid monohydrate and the like; acid addition salt of amines such as pyridine hydrochloride, triethylamine hydrochloride and the like; metal halide such as titanium tetrachloride, zinc chloride, ferrous chloride, ferric chloride and the like; and Lewis acids such as boron trifluoride etherate and the like can be named. These base or acid catalysts can be used normally within a range of 0.001 - equimolar amount to compound (2).

The use ratio of compound (3) to compound (2) is subject to no particular limitation, but it is generally preferred to use 0.5 - 2 mols, in particular 1 - 1.2 mols, of compound (3) per mol of compound (2).

The reaction temperature is variable depending on the kinds of starting materials or that of catalyst used, while normally it can be not higher than the boiling point of the used solvent, preferably within a range of 0 - 110°C.

The reaction of compound (2) with compound (3) can be conducted normally in a solvent inert to the reaction. As the solvent, ethers such as diethyl ether, tetrahydrofuran, dioxane and the like; bipolar aprotic solvents such as N,N-dimethylformamide, dimethylsulfoxide and the like; aromatic hydrocarbons such as benzene, toluene, xylene and the like; nitriles such as acetonitrile and the like; and mixtures of the foregoing can be used.

The reaction time varies depending on the kinds of starting materials and the reaction conditions, while it can be normally around 0.5 - 24 hours.

Compounds (4) which are used as the starting materials in the Method (b) correspond to compounds (1) of the present invention in which R¹⁰ stands for hydrogen, and which can be synthesized, for example, by the above Method (a).

As the halogenating agent to be used for halogenation of compound (4), for example, bromine, chlorine, iodine, N-bromosuccinimide, hydrobromic acid, hydrochloric acid, trichlorobromomethane, sulfuryl chloride and the like can be named. The use rate of these halogenating agent is subject to no particular limitation, while normally convenient range is 0.5 - 2 mols, in particular, 1 - 1.2 mols, per mol of compound (4).

The reaction temperature differs depending on the kind of halogenating agent, while normally it can be not higher than the boiling point of the solvent used, preferably within a range of 0 -110°C.

Halogenation of compound (4) can be conducted normally in a solvent which is inert to the reaction. As the solvent, for example, ethers such as diethyl ether, tetrahydrofuran, dioxane and the like; bipolar aprotic solvents such as N,N-dimethylformamide, dimethylsulfoxide and the like; aromatic hydrocarbons such as benzene; nitriles such as acetonitrile and the like; ketones such as acetone, methyl ethyl ketone and the like; organic acids such as formic acid, acetic acid, propionic acid and the like; carbon halides such as chloroform, carbon tetrachloride, dichloromethane and the like; and mixed solvents of the foregoing can be named.

The reaction time varies depending on the kind of halogenating agent and reaction temperature, while it can be normally around 0.5 - 24 hours.

Compounds (5) which are used as the starting material in the Method (c) correspond to compounds (1) of the present invention wherein R¹⁰ stands for R¹² X, and which can be synthesized by, for example, the above Method (b).

Compounds (6) to be reacted with compounds (5) are per se known compounds, for example, alcohols such as sodium methylate, sodium ethylate, methanol, ethanol, isopropyl alcohol and the like; haloalcohols such as difluoroethanol, trifluoroethanol, trifluoropropanol and the like; mercaptans such as sodium methylmercaptan solution, ethyl mercaptan and the like; and organic acids such as sodium formate, potassium acetate, propionic acid, butanoic acid and the like can be named, which are available on the market.

As the base catalyst useful in the occasion of reacting compound (5) with compound (6), for example, organic bases such as triethylamine, pyridine, 4-N,N-dimethylaminopyridine, N,N-dimethylaniline, 1,4-diazabicyclo[2.2.2]octane, 1,8-diazabicyclo-[5.4.0]undec-7-ene and the like; alkali metal alkoxides such as sodium methoxide, sodium ethoxide, potassium-t-butoxide and the like; inorganic bases such as sodium hydride, potassium hydride, sodium amide, sodium hydroxide, potassium hydroxide, potassium carbonate, sodium carbonate, potassium hydrogencarbonate, sodium hydrogencarbonate and the like; and organometal amides such as lithium diisopropylamide, bistrimethylsilyllithium amide and the like can be named. These base catalysts can be used normally within a range of 0.001 - equimolar amount to compound (5).

Use ratio of compound (6) to compound (5) is not particularly limited, while generally it is preferred to use compound (6) within a range of 0.5 - 2 mols, in particular, 1 - 1.2 mols, per mol of compound (5).

The reaction temperature is variable according to the kinds of starting materials used or kind of the catalyst, while normally it can be no higher than the boiling point of the used solvent, preferably within a range of 0 - 110°C.

The reaction of compound (5) with compound (6) can be conducted normally in a solvent inert to the reaction. As the solvent, ethers such as diethyl ether, tetrahydrofuran, dioxane and the like; bipolar aprotic solvents such as N,N-dimethylformamide, dimethylsulfoxide and the like; aromatic hydrocarbons such as benzene, toluene, xylene and the like; nitriles such as acetonitrile and the like; ketones such as acetone, methyl ethyl ketone and the like; and mixed solvents thereof can be named.

The reaction time varies depending on the kinds of starting materials and the reaction conditions, while it can be normally around 0.5 - 24 hours.

Compounds (1) as obtained in the above Methods (a) - (c) can be isolated and purified by the means known per se, such as, for example, recrystallization, chromatography, distillation or the like.

Compounds of the formula (1) which are provided by the present invention exhibit excellent herbicidal effect as demonstrated by the results of the herbicidal activity tests as shown in later appearing Test Examples 1 - 3, and are useful as herbicidal agents.

Thus, according to the invention, also a weed controlling method characterized by applying effective amount of compound (1) of the present invention to weeds or their habitat is provided.

Compounds (1) of the present invention are active against broad species of agricultural and weeds and are useful as herbicidal agents for paddy field and upland field crops. As paddy field weeds controllable by compounds (1) of the present invention, for example, annual weeds such as barnyard grass (*Echinochloa crusgalli*), Monochoria (*Monochoria vaginalis*), smallflower umbrellaplant *(Cyperus difformis),* Indian toothcup *(Rotala indica),* common falsepimpernel *(Lindernia pyxidaria),* and the like and perennial weeds such as needle spikerush *(Eleocharis acicularis),* Japanese bulrush *(Scirpusjuncoides),* flat sedge *(Cyperus serotinus),* arrowhead *(Sagittaria pygmaea)* and the like can be named. As upland weeds, for example, gramineous weeds such as southern crabgrass *(Digitaria ciliaris),* goosegrass *(Eleusine indica),* green foxtail *(Setaria viridis),* annual bluegrass *(Poa annua)* and the like; and broad-leaved weeds such as common lambsquaters *(Chenopodium album),* slender amaranth *(Amaranthus viridis),* common purslane *(Portulaca oleracea*), tufted knotweed *(Polygonum longisetum*), stickly chickweed *(Cerastium glomeratum*), common groundsel *(Senecio vulgaris),* shepherdspurse (Capsella bursa-pastoris), common chickweed *(Stellaria media)* and the like can be named.

Compounds (1) of the present invention are applicable at any stages of pre- and post-germination of plants, and may furthermore be mixed in the soil before seeding.

The dose of compound (1) of the present invention is variable over a wide range according to kind of the compound, species of the object plant, time of application, site of application and desired effect and the like. As a sort of standard, a range of about 0.01 - 100 g, preferably about 0.1 - 10 g, of the active compound per are can be used by way of example.

Compound (1) of the present invention can be used by itself, but normally it is preferably used as mixed with agriculturally adequate additives such as diluent, surfactant, dispersant, auxiliary and the like according to accepted practice, to formulate the mixtures into such preparation forms as, for example, dust, emulsion, fine granule, granule, wettable powder, granular wettable powder, aqueous suspension, oily suspension, emulsified dispersion, soluble preparation, oily agent, microcapsule and so on.

As examples of solid diluent useful for the formulation, talc, bentonite, montmorillonite, clay, haolin, calcium carbonate, diatomaceous earth, white carbon, vermiculite, slaked lime, siliceous sand, ammonium sulfate, urea and the like can be named.

As liquid diluent, for example, hydrocarbons (e.g., kerosene, mineral oil and the like); aromatic hydrocarbons (e.g., benzene, toluene, xylene, dimethylnaphthalene, phenylxylylethane and the like); chlorinated hydrocarbons (e.g., chloroform, carbon tetrachloride and the like); ethers (e.g., dioxane, tetrahydrofuran and the like); ketones (e.g., acetone, cyclohexanone, isophorone and the like); esters (e.g., ethyl acetate, ethylene glycol acetate, dibutyl maleate and the like; alcohols (e.g., methanol, n-hexanol, ethylene glycol and the like); polar solvents (e.g., N,N-dimethylformamide, dimethylsulfoxide, N-methylpyrrolidone and the like); and water can be named.

As sticking agent and dispersant, for example, casein, polyvinyl alcohol, carboxymethyl cellulose, bentonite, xanthane gum, gum arabic and the like can be named.

As the aerosol propellant, for example, air, nitrogen, carbon dioxide gas, propane, hydrocarbon halide and the like can be named.

As the stabilizer, for example, PAP, BHT and the like can be named.

As the surfactant, for example, alkyl sulfate salt, alkyl sulfonic acid salt, alkylbenzenesulfonic acid salt, lignin sulfonic acid salt, dialkylsulfosuccinic acid salt, naphthalenesulfonic acid salt condensation product, polyoxyethylene alkyl ether, polyoxyethylene alkyl allyl ether, polyoxyethylene alkyl ester, alkylsorbitan ester, polyoxyethylenesorbitan ester, polyoxyethylene alkylamine and the like can be named.

Above-described diluent, surfactant, dispersant and auxiliary can be used each singly or in suitable combination, according to the purpose of use. Furthermore, compounds (1) of the present invention may also be used in combination with other active substances as agricultural chemicals, for example, other herbicide, plant growth regulating agent, fertilizer, insecticide, miticide, fungicide and the like.

Concentration of the active ingredient in the preparations containing compounds (1) of the present invention can be, respectively, normally 1 - 50 wt% in emulsion; normally 0.3 - 25 wt% in dust; normally 1 - 90 wt% in wettable powder and granular wettable powder; normally 0.5 - 10 wt% in granule; normally 0.5 - 40 wt% in suspension; normally 1 - 30 wt% in emulsified dispersion; normally 0.5 - 20 wt% in soluble preparation; and normally 0.1 - 5 wt% in aerosol.

These preparations can be diluted to adequate concentration, where necessary, and sparyed onto plant foliage, soil, water surface of paddy field, or may be applied directly, to serve for various utilities.

### Examples

Hereinafter the present invention is explained still more specifically, referring to working Examples, it being understood that these Examples are not for restricting the scope of the present invention.

### Example 1

### Synthesis of (E)-2-(5-chlorobenzoxazol-2-yl)-1-(3-trifluoromethylphenyl)-3-bromopropene (Compound 1):

To 20 mL of carbon tetrachloride, 0.5 g (1.9 mmols) of (E)-2-(5-chlorobenzoxazol-2-yl)-1-(3-trifluoromethylphenyl)propene, 0.3 g (1.9 mmols) of N-bromosuccinimide and 0.1 g of benzoyl peroxide were added and stirred under reflux for 3 hours. Cooling the system to room temperature, the organic layer was washed with saturated saline and dried over anhydrous sodium sulfate. Distilling the carbon tetrachloride off under reduced pressure, the resulting residue was isolated on column chromatography (Wakogel C-300, Wako Pure Chemical Industries, Ltd.; n-hexane: ethyl acetate = 15:1 elution) to provide 0.5 g of the object Compound 1 as pale yellow crystals.

### Example 2

### Synthesis of (E)-2-(5-chlorobenzoxazol-2-yl)-1-(3-trifluoromethylphenyl)-3-methoxymethylpropene (Compound 3):

To 20 mL of xylene, 0.5 g (1.9 mmols) of 2-amino-4-chlorophenol, 0.50 g (1.9 mmols) of (E)-α-methoxymethyl-(3-trifluoromethyl)cinnamic acid chloride and 0.1 g of p-toluenesulfonic acid monohydrate were added and refluxed for 8 hours. Cooling the system to room temperature, the xylene was distilled off under reduced pressure, and the resulting residue was isolated on column chromatography (Wakogel C-300, Wako Pure Chemical Industries, Ltd.; n-hexane: ethyl acetate = 15:1 elution) to provide 0.72 g of the object Compound 3 as pale yellow crystals (yield = 60%).

### Example 3

### Synthesis of (E)-2-(5-chlorobenzoxazol-2-yl)-1-(3-trifluoromethylphenyl)-3-ethoxymethylpropene (Compound 11):

In 20 mL of ethanol, 0.5 g (1.9 mmols) of (E)-2-(5-chloro-benzoxazol-2-yl)-1-(3-trifluoromethylphenyl)-3-bromopropene and 0.2 g (1.9 mmols) of sodium ethoxide were stirred at room temperature for 3 hours. Thirty (30) mL of toluene was added to the reaction liquid and the organic layer was washed with water and saturated saline, and dried over anhydrous sodium sulfate. Distilling the toluene off under reduced pressure, the resulting residue was isolated on column chromatography (Wakogel C-300, Wako Pure Chemical Industries, Ltd.; n-hexane: ethyl acetate = 15:1 elution) to provide 0.5 g of the object Compound 11 as pale yellow crystals.

### Example 4

### Synthesis of (E)-2-(5-chlorobenzoxazol-2-yl)-1-(3-trifluoromethylphenyl)-3-formylpropene (Compound 33):

In 20 mL of N,N-dimethylformamide, 0.5 g (1.9 mmols) of (E)-2-(5-chlorobenzoxazol-2-yl)-1-(3-trifluoromethylphenyl)-3-bromopropene, 0.2 g (1.9 mmols) of potassium formate and 0.04 g of 18-crown-6 were heated and stirred for 8 hours at 80°C. Cooling the system to room temperature, 30 mL of toluene was added. The organic layer was washed with water and saturated saline and dried over anhydrous sodium sulfate. Distilling the toluene off under reduced pressure, the resulting residue was isolated on column chromatography (Wakogel C-300, Wako Pure Chemical Industries, Ltd.; n-hexane: ethyl acetate = 15:1 elution) to provide 0.5 g of the object Compound 33 as pale yellow crystals.

### Example 5

### Synthesis of (E)-2-(5-trifluoromethylbenzoxazol-2-yl)-1-(3-trifluoromethylphenyl)-3-(2,2,2-trifluoroethoxy)propene (Compound 85):

In 20 mL of tetrahydrofuran, 0.2 g (1.9 mmols) of 2,2,2-trifluoroethanol and 0.1 g (2.5 mmols) of 60% sodium hydride were stirred at room temperature for an hour, into which 0.5 g (1.9 mmols) of (E)-2-(5-trifluoromethylbenzoxazol-2-yl)-1-(3-trifluoromethylphenyl)-3-bromopropene was added, followed by 5 hours' stirring at room temperature. Thirty (30) mL of toluene was added to the reaction liquid, and the organic layer was washed with water and saturated saline, and dried over anhydrous sodium sulfate. Distilling the toluene off under reduced pressure, the resulting residue was isolated on column chromatography (Wakogel C-300, Wako Pure Chemical Industries, Ltd.; n-hexane: ethyl acetate = 15:1 elution) to provide 0.5 g of the object Compound 85 as pale yellow crystals.

Following the methods as described in above Examples 1 - 5, other compounds of the present invention as identified in the following Table 1 were synthesized. The synthesized compounds (1) inclusive of the compounds which were synthesized in Examples 1 - 5 are shown in Table 1, and physical properties of the representative of those compounds are shown in Table 2.

**TABLE 1**

| | | | | | | | | | | |
|---|---|---|---|---|---|---|---|---|---|---|
| 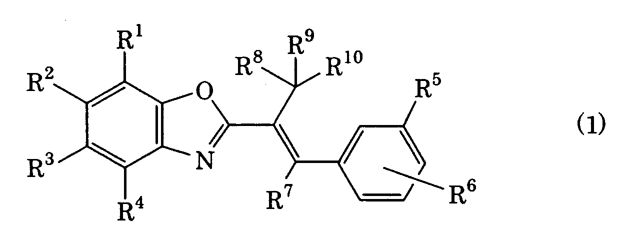 | | | | | | | | | | |

| Compound | R¹ | R² | R³ | R⁴ | R⁵ | R⁶ | R⁷ | R⁸ | R⁹ | R¹⁰ |
|---|---|---|---|---|---|---|---|---|---|---|
| 1 | H | H | Cl | H | CF₃ | H | H | H | H | Br |
| 2 | H | H | Cl | H | CF₃ | H | H | H | H | CN |
| 3 | H | H | Cl | H | CF₃ | H | H | H | H | OCH₃ |
| 4 | H | H | Cl | H | CF₃ | 5-CF₃ | H | H | H | OCH₃ |
| 5 | H | H | Cl | H | CF₃ | 5-CF₃ | H | H | H | OC₂H₅ |
| 6 | H | H | Cl | H | CN | H | H | H | H | OCH₃ |
| 7 | H | H | Cl | CH₃ | CF₃ | H | H | H | H | OCH₃ |
| 8 | H | H | Cl | H | CF₃ | 4-F | H | H | H | OCH₃ |
| 9 | H | H | Cl | H | CF₃ | 4-Cl | H | H | H | OCH₃ |
| 10 | H | H | Cl | H | CF₃ | 4-CN | H | H | H | OCH₃ |
| 11 | H | H | Cl | H | CF₃ | H | H | H | H | OC₂H₅ |
| 12 | H | H | Cl | H | CF₃ | H | CH₃ | H | H | OCH₃ |
| 13 | H | H | Cl | H | CF₃ | 4-F | CH₃ | H | H | OCH₃ |
| 14 | H | H | Cl | H | CF₃ | H | CH₃ | H | H | OC₂H₅ |
| 15 | H | H | Cl | H | CF₃ | 4-F | CH₃ | H | H | OC₂H₅ |
| 16 | H | H | Cl | H | CF₃ | H | C₂H₅ | H | H | OCH₃ |
| 17 | H | H | Cl | H | CF₃ | H | C₂H₅ | H | H | OC₂H₅ |
| 18 | H | H | Cl | H | CF₃ | H | n-C₃H₇ | H | H | OCH₃ |
| 19 | H | H | Cl | H | CF₃ | H | n-C₃H₇ | H | H | OC₂H₅ |
| 20 | H | H | Cl | H | CF₃ | H | n-C₄H₉ | H | H | OCH₃ |
| 21 | H | H | Cl | H | CF₃ | H | n-C₄H₉ | H | H | OC₂H₅ |
| 22 | H | H | Cl | H | CF₃ | H | Cl | H | H | OCH₃ |
| 23 | H | H | Cl | H | CF₃ | 4-F | Cl | H | H | OCH₃ |
| 24 | H | H | Cl | H | CF₃ | H | Br | H | H | OCH₃ |
| 25 | H | H | Cl | H | CF₃ | 4-F | Br | H | H | OCH₃ |
| 26 | H | H | Cl | H | CF₃ | 4-F | H | H | H | OC₂H₅ |
| 27 | H | H | Cl | H | CF₃ | H | H | H | H | OC₃H₇ |
| 28 | H | H | Cl | H | CF₃ | 4-CN | H | H | H | OC₃H₇ |
| 29 | H | H | Cl | H | CF₃ | H | H | H | H | O-i-C₃H₇ |
| 30 | H | H | Cl | H | CF₃ | H | H | H | H | SCH₃ |
| 31 | H | H | Cl | H | CF₃ | 4-F | H | H | H | SCH₃ |
| 32 | H | H | Cl | H | CF₃ | H | H | H | H | SC₂H₅ |
| 33 | H | H | Cl | H | CF₃ | H | H | H | H | OCOH |
| 34 | H | H | F | H | CF₃ | H | H | H | H | OCH₃ |
| 35 | H | H | F | H | CF₃ | 4-F | H | H | H | OC₂H₅ |
| 36 | H | H | F | H | CF₃ | 4-CN | H | H | H | OCH₃ |
| 37 | H | H | F | H | CF₃ | H | H | CH₃ | H | OCH₃ |
| 38 | H | H | F | H | CF₃ | H | H | H | H | SCH₃ |
| 39 | H | H | Br | H | CF₃ | H | H | H | H | OCH₃ |
| 40 | H | H | Br | H | CF₃ | 4-F | H | H | H | OC₂H₅ |
| 41 | H | H | I | H | CF₃ | H | H | R H | H | OCH₃ |
| 42 | H | H | I | H | CF₃ | 4-F | H | H | H | OCH₃ |
| 43 | H | H | CN | H | CF₃ | H | H | H | H | OCH₃ |
| 44 | H | H | CN | H | CF₃ | 4-F | H | H | H | OC₂H₅ |
| 45 | H | H | NO₂ | H | CF₃ | H | H | H | H | OCH₃ |
| 46 | H | H | OCF₃ | H | CF₃ | H | H | H | H | OCH₃ |
| 47 | H | H | CO₂H | H | CF₃ | H | H | H | H | OCH₃ |
| 48 | H | H | CO₂CH₃ | H | CF₃ | H | H | H | H | OCH₃ |
| 49 | H | H | CO₂C₂H₅ | H | CF₃ | H | H | H | H | OCH₃ |
| 50 | H | H | CO₂-n-C₃H₇ | H | CF₃ | H | H | H | H | OCH₃ |
| 51 | H | H | CO₂-n-C₄H₉ | H | CF₃ | H | H | H | H | OCH₃ |
| 52 | H | H | CF₃ | H | CF₃ | H | H | H | H | OCH₃ |
| 53 | H | H | CF₃ | H | CF₃ | 4-F | H | H | H | OCH₃ |
| 54 | H | H | CF₃ | H | CF₃ | H | H | H | H | OC₂H₅ |
| 55 | H | H | CF₃ | H | CF₃ | 4-F | H | H | H | OC₂H₅ |
| 56 | H | H | CF₃ | H | CF₃ | H | H | H | H | O-n-C₃H₇ |
| 57 | H | H | CF₃ | H | CF₃ | 4-F | H | H | H | O-n-C₃H₇ |
| 58 | H | H | CF₃ | H | CF₃ | H | H | H | H | O-n-C₄H₉ |
| 59 | H | H | CF₃ | H | CF₃ | 4-F | H | H | H | O-n-C₄H₉ |
| 60 | H | H | CF₃ | H | CF₃ | H | H | H | H | O-n-C₆H₁₃ |
| 61 | H | H | CF₃ | H | CF₃ | 4-F | H | H | H | O-n-C₆H₁₃ |
| 62 | H | H | CF₃ | H | CF₃ | H | H | H | H | O-n-C₈H₁₇ |
| 63 | H | H | CF₃ | H | CF₃ | 4-F | H | H | H | O-n-C₈H₁₇ |
| 64 | H | H | CF₃ | H | CF₃ | H | H | H | H | O-n-C₁₀H₂₁ |
| 65 | H | H | CF₃ | H | CF₃ | 4-F | H | H | H | O-n-C₁₀H₂₁ |
| 66 | H | H | CF₃ | H | CF₃ | H | H | H | H | O-cyclo-C₅H₉ |
| 67 | H | H | CF₃ | H | CF₃ | 4-F | H | H | H | O-cyclo-C₅H₉ |
| 68 | H | H | CF₃ | H | CF₃ | H | H | H | H | O₋cyclo-C₆H₁₁ |
| 69 | H | H | CF₃ | H | CF₃ | 4-F | H | H | H | O-cyclo-C₆H₁₁ |
| 70 | H | H | CF₃ | H | CF₃ | H | H | CH₃ | H | OCH₃ |
| 71 | H | H | CF₃ | H | CF₃ | 4-F | H | CH₃ | H | OC₂H₅ |
| 72 | H | H | CF₃ | H | CF₃ | H | H | C₂H₅ | H | OCH₃ |
| 73 | H | H | CF₃ | H | CF₃ | 4-F | H | C₂H₅ | H | OC₂H₅ |
| 74 | H | H | CF₃ | H | CF₃ | H | H | n-C₄H₉ | H | OCH₃ |
| 75 | H | H | CF₃ | H | CF₃ | 4-F | H | n-C₄H₉ | H | OC₂H₅ |
| 76 | H | H | CF₃ | H | CF₃ | H | H | CH₃ | CH₃ | OCH₃ |
| 77 | H | H | CF₃ | H | CF₃ | 4-F | H | CH₃ | CH₃ | OC₂H₅ |
| 78 | H | H | CF₃ | H | CF₃ | H | H | C₂H₅ | CH₃ | OCH₃ |
| 79 | H | H | CF₃ | H | CF₃ | 4-F | H | C₂H₅ | CH₃ | OC₂H₅ |
| 80 | H | H | CF₃ | H | CF₃ | H | H | n-C₄H₉ | CH₃ | OCH₃ |
| 81 | H | H | CF₃ | H | CF₃ | 4-F | H | R n-C₄H₉ | CH₃ | R¹⁰ OC₂H₅ |
| 82 | H | H | CF₃ | H | CF₃ | H | H | H | H | SCH₃ |
| 83 | H | H | CF₃ | H | CF₃ | 4-F | H | H | H | SCH₃ |
| 84 | H | H | CF₃ | H | CF₃ | H | H | H | H | SC₂H₅ |
| 85 | H | H | CF₃ | H | CF₃ | H | H | H | H | OCH₂CF₃ |
| 86 | H | H | CF₃ | H | CF₃ | 4-F | H | H | H | OCH₂CF₃ |
| 87 | H | H | CF₃ | H | CF₃ | H | H | H | H | OCH₂CF₂CF₂H |
| 88 | H | H | CF₃ | H | CF₃ | 4-F | H | H | H | OCH₂CF₂CF₂H |
| 89 | H | H | CF₃ | H | CF₃ | H | H | H | H | OCH₂CH₂Cl |
| 90 | H | H | CF₃ | H | CF₃ | 4-F | H | H | H | OCH₂CH₂Cl |
| 91 | H | H | CF₃ | H | CF₃ | H | H | H | H | O(CH₂)₄Cl |
| 92 | H | H | CF₃ | H | CF₃ | 4-F | H | H | H | O(CH₂)₄Cl |
| 93 | H | H | CF₃ | H | CF₃ | H | H | H | H | O(CH₂)₆Cl |
| 94 | H | H | CF₃ | H | CF₃ | 4-F | H | H | H | O(CH₂)₆Cl |
| 95 | H | H | CF₃ | H | CF₃ | H | H | H | H | OCOH |
| 96 | H | H | CF₃ | H | CF₃ | 4-F | H | H | H | OCOH |
| 97 | H | H | CF₃ | H | CF₃ | H | H | H | H | OCOCH₃ |
| 98 | H | H | CF₃ | H | CF₃ | 4-F | H | H | H | OCOCH₃ |
| 99 | H | H | CF₃ | H | CF₃ | H | H | H | H | OCOC₂H₅ |
| 100 | H | H | CF₃ | H | CF₃ | 4-F | H | H | H | OCOC₂H₅ |
| 101 | H | H | CF₃ | H | CF₃ | H | H | H | H | OCO-n-C₄H₉ |
| 102 | H | H | CF₃ | H | CF₃ | 4-F | H | H | H | OCO-n-C₄H₉ |
| 103 | H | H | CF₃ | H | CF₃ | H | H | H | H | OCO-n-C₆H₁₃ |
| 104 | H | H | CF₃ | H | CF₃ | 4-F | H | H | H | OCO-n-C₆H₁₃ |
| 105 | CH₃ | H | Cl | H | CF₃ | H | H | H | H | OCH₃ |
| 106 | Cl | H | Cl | H | CF₃ | H | H | H | H | OCH₃ |
| 107 | Cl | H | Cl | H | CF₃ | 4-F | H | H | H | OC₂H₅ |
| 108 | Cl | H | Cl | H | CF₃ | H | H | H | H | OC₂H₅ |
| 109 | F | H | F | H | CF₃ | H | H | H | H | OCH₃ |
| 110 | F | H | F | H | CF₃ | 4-F | H | H | H | OC₂H₅ |
| 111 | F | H | F | H | CF₃ | 4-F | H | H | H | SCH₃ |
| 112 | F | H | F | H | CF₃ | H | H | H | H | OC₂H₅ |
| 113 | F | H | F | H | CF₃ | H | H | H | H | OC₃H₇ |
| 114 | F | H | Cl | H | CF₃ | H | H | H | H | OCH₃ |
| 115 | F | H | Cl | H | CF₃ | 4-F | H | H | H | OC₂H₅ |
| 116 | H | F | Cl | H | CF₃ | H | H | H | H | OCH₃ |
| 117 | H | F | CN | H | CF₃ | H | H | H | H | OCH₃ |
| 118 | H | F | NO₂ | H | CF₃ | H | H | H | H | OCH₃ |
| 119 | H | Cl | Cl | H | CF₃ | H | H | H | H | OCH₃ |
| 120 | H | Cl | Cl | H | CF₃ | 4-F | H | H | H | OCH₃ |
| 121 | Cl | H | H | Cl | CF₃ | H | H | H | H | OCH₃ OCH₃ |
| 122 | Cl | H | H | Cl | CF₃ | 4-F | H | H | H | OCH₃ |
| 123 | Cl | H | H | Cl | CF₃ | H | H | H | H | OC₂H₅ |
| 124 | Cl | H | H | Cl | CF₃ | 4-F | H | H | H | OC₂H₅ |
| 125 | H | F | F | H | CF₃ | H | H | H | H | OCH₃ |
| 126 | H | F | F | H | CF₃ | 4-F | H | H | H | OCH₃ |
| 127 | H | F | F | H | CF₃ | H | H | H | H | OC₂H₅ |
| 128 | F | H | H | F | CF₃ | H | H | H | H | OCH₃ |
| 129 | F | H | H | F | CF₃ | 4-F | H | H | H | OCH₃ |
| 130 | F | H | H | F | CF₃ | H | H | H | H | OC₂H₅ |
| 131 | F | H | H | F | CF₃ | 4-F | H | H | H | OC₂H₅ |
| 132 | H | Cl | CN | H | CF₃ | H | H | H | H | OCH₃ |
| 133 | H | Cl | F | H | CF₃ | H | H | H | H | OCH₃ |
| 134 | H | Cl | NO₂ | H | CF₃ | H | H | H | H | OCH₃ |
| 135 | H | H | SOCH₃ | H | CF₃ | H | H | H | H | OCH₃ |
| 136 | H | H | SO₂CH₃ | H | CF₃ | H | H | H | H | OCH₃ |
| 137 | H | H | SO-t-C₄H₉ | H | CF₃ | H | H | H | H | OCH₃ |
| 138 | H | H | SO₂-t-C₄H₉ | H | CF₃ | H | H | H | H | OCH₃ |
| 139 | H | CF₃ | F | H | CF₃ | H | H | H | H | OCH₃ |
| 140 | H | CF₃ | F | H | CF₃ | 4-F | H | H | H | OCH₃ |
| 141 | H | CF₃ | F | H | CF₃ | H | H | H | H | OC₂H₅ |
| 142 | H | CF₃ | F | H | CF₃ | 4-F | H | H | H | OC₂H₅ |
| 143 | t-C₄H₉ | H | H | H | CF₃ | H | H | H | H | OCH₃ |
| 144 | t-C₄H₉ | H | H | H | CF₃ | 4-F | H | H | H | OCH₃ |
| 145 | t-C₄H₉ | H | H | H | CF₃ | H | H | H | H | OC₂H₅ |
| 146 | t-C₄H₉ | H | H | H | CF₃ | 4-F | H | H | H | OC₂H₅ |
| 147 | H | t-C₄H₉ | H | H | CF₃ | H | H | H | H | OCH₃ |
| 148 | H | t-C₄H₉ | H | H | CF₃ | 4-F | H | H | H | OCH₃ |
| 149 | H | t-C₄H₉ | H | H | CF₃ | H | H | H | H | OC₂H₅ |
| 150 | H | t-C₄H₉ | H | H | CF₃ | 4-F | H | H | H | OC₂H₅ |
| 151 | H | H | t-C₄H₉ | H | CF₃ | H | H | H | H | OCH₃ |
| 152 | H | H | t-C₄H₉ | H | CF₃ | 4-F | H | H | H | OCH₃ |
| 153 | H | H | t-C₄H₉ | H | CF₃ | H | H | H | H | OC₂H₅ |
| 154 | H | H | t-C₄H₉ | H | CF₃ | 4-F | H | H | H | OC₂H₅ |
| 155 | H | H | OCF₃ | H | CF₃ | H | H | H | H | OCH₃ |
| 156 | H | H | OCF₃ | H | CF₃ | H | H | H | H | OC₂H₅ |
| 157 | H | H | OCF₃ | H | CF₃ | 4-F | H | H | H | OCH₃ |
| 158 | H | H | OCF₃ | H | CF₃ | 4-F | H | H | H | OC₂H₅ |
| 159 | H | H | OCH₂CF₃ | H | CF₃ | H | H | H | H | OCH₃ |
| 160 | H | H | OCH₂CF₃ | H | CF₃ | H | H | H | H | OC₂H₅ |
| 161 | H | H | OCH₂CF₃ | H | CF₃ | 4-F | H | H | H | OCH₃ |
| 162 | H | H | OCH₂CF₃ | H | CF₃ | 4-F | H | H | H | OC₂H₅ |
| 163 | H | H | O(CH₂)₄Cl | H | CF₃ | H | H | H | H | OCH₃ |
| 164 | H | H | O(CH₂)₄Cl | H | CF₃ | H | H | H | H | OC₂H₅₃ |
| 165 | H | H | O(CH₂)₄Cl | H | CF₃ | 4-F | H | H | H | OCH₃ |
| 166 | H | H | O(CH₂)₄Cl | H | CF₃ | 4-F | H | H | H | OC₂H₅ |
| 167 | H | H | CHO | H | CF₃ | H | H | H | H | OCH₃ |
| 168 | H | H | CHO | H | CF₃ | H | H | H | H | OC₂H₅ |
| 169 | H | H | CHO | H | CF₃ | 4-F | H | H | H | OCH₃ |
| 170 | H | H | CHO | H | CF₃ | 4-F | H | H | H | OC₂H₅ |
| 171 | H | H | COCH₃ | H | CF₃ | H | H | H | H | OCH₃ |
| 172 | H | H | COCH₃ | H | CF₃ | H | H | H | H | OC₂H₅ |
| 173 | H | H | COCH₃ | H | CF₃ | 4-F | H | H | H | OCH₃ |
| 174 | H | H | COCH₃ | H | CF₃ | 4-F | H | H | H | OC₂H₅ |
| 175 | H | H | CO-t-Bu | H | CF₃ | H | H | H | H | OCH₃ |
| 176 | H | H | CO-t-Bu | H | CF₃ | H | H | H | H | OC₂H₅ |
| 177 | H | H | CO-t-Bu | H | CF₃ | 4-F | H | H | H | OCH₃ |
| 178 | H | H | CO-t-Bu | H | CF₃ | 4-F | H | H | H | OC₂H₅ |
| 179 | H | F | CN | H | CF₃ | H | H | H | H | OC₂H₅ |
| 180 | H | CF₃ | H | H | CF₃ | H | H | H | H | OCH₃ |
| 181 | H | CF₃ | H | H | CF₃ | H | H | H | H | OC₂H₅ |

**TABLE 2**

| Compound | H1NMR (60MHz, CDCl3, δ(ppm)) | Melting point (°C) |
|---|---|---|
| 1 | 4.62 (2H, s), 7.1-8.0 (8H, m) | 151-152.5 |
| 3 | 3.50 (3H, s), 4.38 (2H, s), 7.1-8.0 (8H, m) | 100-103 |
| 9 | 3.61 (3H, s), 4.57 (2H, s), 7.1-8.1 (7H, m) | 147-150 |
| 11 | 1.30 (3H, t, J=7.0), 3.70 (2H, q, J=7.0), 4.53 (2H, s), 7.0-8.1 (8H, m) | 107-111 |
| 27 | 0.96 (3H, t, J=6.0), 1.4-2.0 (2H, m), 3.58 (2H, t, J=6.2), 4.52 (2H, s), 7.0-8.1 (8H, m) | 93-97 |
| 29 | 1.33 (6H, d, J=6.0), 3.5-4.2 (1H, m), 7.0-8.1 (8H, m) | 76-78 |
| 30 | 2.23 (3H, s), 3.90 (2H, s), 7.1-8.0 (8H, m) | 114-117 |
| 32 | 1.23 (3H, t, J=7.0), 2.72 (2H, q, J=7.0), 3.93 (2H, s), 7.1-8.1 (8H, m) | 97-101 |
| 33 | 5.33 (2H, s), 7.2-8.3 (9H, m) | 140-143 |
| 34 | 3.51 (3H, s), 4.47 (2H, s), 6.8-8.0 (8H, m) | 103-106 |
| 39 | 3.52 (3H, s), 4.50 (2H, s), 7.1-8.2 (8H, m) | 70-73 |
| 43 | 3.55 (3H, s), 4.52 (2H, s), 7.1- 8.2 (8H, m) | 156-158 |
| 46 | 3.53 (3H, s), 4.52 (2H, s), 7.0-8.1 (8H, m) | 115-117 |
| 52 | 3.56 (3H, s), 4.56 (2H, s), 7.3-8.2 (8H, m) | 103-106 |
| 54 | 1.38 (3H, t,J=7.0), 3.77 (2H, q, J=7.0), 4.58 (2H, s), 7.2-8.2 (8H, m) | 75-79 |
| 82 | 2.23 (3H, s), 3.94 (2H, s), 7.2-8.2 (8H, m) | 97-101 |
| 84 | 1.28 (3H, t, J=7.0), 2.72 (2H, t, J=7.0), 3.95 (2H, s), 7.1-8.1 (8H, m) | 77-80 |
| 85 | 4.10 (2H, q, J=8.5), 4.77 (2H, s), 7.2-8.2 (8H, m) | 86-89 |
| 87 | 4.10 (2H, tt, J1=13.0, J2=1.4), 4.77 (2H, s), 5.99 (1H, tt, J1=53.0, J2=5.0), 7.1-8.2 (8H, m) | 86-89 |
| 89 | 3.5-4.1 (4H, m), 3.5-4.2 (1H, m), 7.0-8.1 (8H, m) | 96-98 |
| 106 | 3.52 (3H, s), 4.49 (2H, s), 7.2-8.2 (7H, m) | 150-155 |
| 108 | 1.30 (3H, t, J=7.0), 3.72 (2H, q, J=7.0), 7.1-8.1 (7H, m) | 111-115 |
| 109 | 3.53 (3H, s), 4.50 (2H, s), 6.6-8.1 (7H, m) | 122-125 |
| 112 | 1.31 (3H, t, J=7.0), 3.66 (2H, q, J=7.0), 4.52 (2H, s), 6.6-8.1 (7H, m) | 80-84 |
| 117 | 3.56 (3H, s), 4.52 (2H, s), 7.2-8.2 (7H, m) | 133-134 |
| 119 | 3.53 (3H, s), 4.48 (2H, s), 7.3-8.1 (7H, m) | 126-129 |
| 125 | 3.52 (3H, s), 4.48 (2H, s), 7.2-8.0 (7H, m) | 123-127 |
| 126 | 3.55 (3H, s), 4.50 (2H, s), 7.1-8.0 (6H, m) | 131-133 |
| 127 | 1.32 (3H, t, J=7.0), 3.72 (2H, q, J=7.0), 4.50 (2H, s), 7.1-8.1 (7H, m) | 83-87 |
| 141 | 1.33 (3H, t,J=7.0), 3.70 (2H, q, J=7.0), 4.56 (2H, s), 7.2-8.2 (7H, m) | 79-82 |
| 151 | 1.33 (9H, s), 3.53 (2H, s), 4.54 (2H, s), 7.0-8.1 (8H, m) | 74-78 |
| 156 | 0.95 (3H, t, J=6.0), 1.4-2.0 (2H, m), 3.59 (2H, t, J=6.2), 4.54 (2H, s), 6.9-8.2 (8H, m) | 86-89 |
| 171 | 2.11 (3H, s), 3.56 (3H, s), 4.62 (2H, s), 7.2-8.4 (8H, m) | 105-107 |

### Preparation Example 1

### Preparation of granule

Five (5) wt parts of Compound 1,35 wt parts of bentonite, 57 wt parts of talc, 1 wt part of sodium dodecylbenzenesulfonate and 2 wt parts of sodium lignosulfonate were uniformly mixed. Then a minor amount of water was added and kneaded together, followed by granulation by extrusion and drying to provide a granule.

### Preparation Example 2

### Preparation of wettable powder

A wettable powder was obtained by uniformly mixing 10 wt parts of Compound 1, 70 wt parts of kaolin clay, 18 wt parts of white carbon, 1.5 wt parts of sodium dodecylbenzenesulfonate and 0.5 wt part of sodium β-naphthalenesulfonate-formaline condensation product; and air mill grinding the mixture.

### Preparation Example 3

### Preparation of emulsion

An emulsion was obtained by adding 10 wt parts of SORPOL 3005 X (tradename; Toho Chemical Industry Co. Ltd) to 20 wt parts of Compound 1 and 70 wt parts of xylene, uniformly mixing and dissolving them.

### Preparation Example 4

### Preparation of dust

A dust was obtained by uniformly mixing 5 wt parts of Compound 1, 50 wt parts of talc and 45 wt parts of kaolin clay.

### [Herbicidal activity test]

### Test Example 1

### Weed control test for paddy field

Paddy field soil was filled in 1/10,000 are pots, kneaded with adequate amounts of water and chemical fertilizer, seeded with barnyard grass (*Echinochloa crusgalli*), Monochoria (*Monochoria vaginalis*), Japanese bulrush (*Scirpus juncoides*) and flat sedge (*Cyperus serotinus*), and further transplanted with 2.0 leaf stage of rice plant. The pots were maintained under the water-filled condition to a depth of 3 cm.

Wettable powders containing the compounds (1) as identified in Table 1, which were formulated following Production Example 2, were diluted with adequate amount of water and applied to the pots dropwise with pippet at a prescribed dose of the active ingredients per are, at 1.0 leaf stage of the barnyard grass.

After placing the pots in a glasshouse which was controlled at an average temperature of 25°C for 3 weeks, herbicidal effect was examined.

Evaluation of the herbicidal effect is shown in the following six grades, by comparing the condition of untreated control and growth inhibition (%):
0 : 0% - less than 20%
1 : 20% - less than 40%
2 : 40% - less than 60%
3 : 60% - less than 80%
4 : 80% - less than 100%
5 : 100%.

Evaluation of phytotoxicity is shown in the following six grades, as compared with the condition of untreated control:
0 : normal growth
1 : little damaged
2 : low damaged
3 : medium damaged
4 : serious damaged
5 : complete killed.
The results were as shown in Table 3.

**TABLE 3**

| | | Treated at 1.0 leaf stage of barnyard grass | | | | |
|---|---|---|---|---|---|---|
| | | Herbicidal Effect | | | | Phytotoxicity |
| Compound No | Dose (g^{ai}/a) a | Barnyard grass | Monochoria vaginalis | Japanese bulrush | Flat sedge | Rice plant |
| 3 | 2.5 | 5 | 5 | 4 | 3 | 1 |
| | 5 | 5 | 5 | 5 | 5 | 1 |
| 9 | 2.5 | 4 | 5 | 3 | 2 | 0 |
| | 5 | 5 | 5 | 4 | 3 | 0 |
| 11 | 2.5 | 5 | 5 | 4 | 3 | 1 |
| | 5 | 5 | 5 | 5 | 5 | 1 |
| 30 | 2.5 | 4 | 5 | 4 | 3 | 0 |
| | 5 | 5 | 5 | 5 | 5 | 0 |
| 39 | 2.5 | 5 | 5 | 3 | 2 | 0 |
| | 5 | 5 | 5 | 4 | 3 | 0 |
| 52 | 2.5 | 5 | 5 | 4 | 3 | 0 |
| | 5 | 5 | 5 | 5 | 4 | 1 |
| 54 | 2.5 | 5 | 5 | 4 | 3 | 1 |
| | 5 | 5 | 5 | 5 | 4 | 1 |
| 85 | 2.5 | 4 | 5 | 4 | 3 | 0 |
| | 5 | 5 | 5 | 5 | 4 | 0 |
| 112 | 2.5 | 4 | 5 | 4 | 3 | 0 |
| | 5 | 5 | 5 | 5 | 4 | 1 |
| 126 | 2.5 | 5 | 5 | 3 | 2 | 0 |
| | 5 | 5 | 5 | 4 | 3 | 0 |
| 151 | 2.5 | 4 | 5 | 3 | 3 | 0 |
| | 5 | 5 | 5 | 5 | 4 | 0 |

### Test Example 2

### Soil treatment test for upland field

Upland field soil was filled in 1/3,000 are pots, seeded with southern crabgrass (*Digitaria ciliaris*), common lambsquaters (*Chenopodium album*), slender amaranth (*Amaranthus viridis*), common purslane (*Portulaca oleracea*), velvetleaf (*Abutilon theophrasti*), wheat, corn and soybean. The seeds were covered up with the soil.

Wettable powders containing the object compounds (1) as identified in Table 1, which were formulated following Preparation Example 2, were diluted with adequate amount of water to secure the prescribed dose of the active ingredient, and uniformly sprayed onto the surface soil layer after the seeding and before germination of the weeds, at an application water of 15 liters per are.

After placing the test pots in a glasshouse which was kept at an average temperature of 25°C for 3 weeks, the herbicidal effect was examined.

Evaluations of the herbicidal effect and phytotoxicity were made in the manner similar to those in the above Test Example 1. The results were as shown in Table 4.

**TABLE 4**

| | | Soil Treatment | | | | | | | |
|---|---|---|---|---|---|---|---|---|---|
| | | Herbicidal Effect | | | | | Phytotoxicity | | |
| Compound No. | Dose (gai/a) | southern crabgrass | common lamb-squaters | slender amaranth | common purslane | velvetleaf | wheat | corn | soybean |
| 3 | 2.5 | 5 | 5 | 5 | 5 | 3 | 0 | 0 | 0 |
| | 5 | 5 | 5 | 5 | 5 | 4 | 0 | 1 | 0 |
| 9 | 2.5 | 5 | 5 | 5 | 5 | 4 | 0 | 1 | 0 |
| | 5 | 5 | 5 | 5 | 5 | 5 | 1 | 1 | 0 |
| 11 | 2.5 | 5 | 5 | 5 | 5 | 3 | 0 | 1 | 0 |
| | 5 | 5 | 5 | 5 | 5 | 4 | 0 | 1 | 0 |
| 30 | 2.5 | 3 | 4 | 5 | 5 | 2 | 0 | 0 | 0 |
| | 5 | 5 | 4 | 5 | 5 | 2 | 1 | 0 | 0 |
| 39 | 2.5 | 4 | 5 | 5 | 5 | 4 | 0 | 0 | 0 |
| | 5 | 5 | 5 | 5 | 5 | 5 | 0 | 1 | 1 |
| 46 | 2.5 | 4 | 5 | 5 | 5 | 4 | 0 | 0 | 0 |
| | 5 | 5 | 5 | 5 | 5 | 5 | 0 | 1 | 1 |
| 52 | 2.5 | 5 | 5 | 5 | 5 | 5 | 1 | 1 | 0 |
| | 5 | 5 | 5 | 5 | 5 | 5 | 1 | 1 | 1 |
| 54 | 2.5 | 5 | 5 | 5 | 5 | 5 | 1 | 1 | 0 |
| | 5 | 5 | 5 | 5 | 5 | 5 | 1 | 1 | 1 |
| 82 | 2.5 | 3 | 5 | 5 | 5 | 2 | 0 | 0 | 0 |
| | 5 | 3 | 5 | 5 | 5 | 2 | 0 | 0 | 0 |
| 84 | 2.5 | 3 | 5 | 4 | 5 | 2 | 0 | 0 | 0 |
| | 5 | 3 | 5 | 5 | 5 | 2 | 0 | 0 | 0 |
| 85 | 2.5 | 5 | 5 | 5 | 5 | 5 | 0 | 0 | 0 |
| | 5 | 5 | 5 | 5 | 5 | 5 | 0 | 0 | 0 |
| 87 | 2.5 | 4 | 5 | 5 | 5 | 4 | 0 | 0 | 0 |
| | 5 | 5 | 5 | 5 | 5 | 5 | 1 | 0 | 1 |
| 126 | 2.5 | 4 | 5 | 5 | 5 | 3 | 0 | 0 | 0 |
| | 5 | 5 | 5 | 5 | 5 | 4 | 0 | 0 | 0 |
| 127 | 2.5 | 4 | 5 | 5 | 5 | 3 | 0 | 0 | 0 |
| | 5 | 5 | 5 | 5 | 5 | 5 | 0 | 0 | 0 |
| 141 | 2.5 | 4 | 5 | 5 | 5 | 3 | 0 | 0 | 0 |
| | 5 | 5 | 5 | 5 | 5 | 5 | 1 | 0 | 0 |

### Test Example 3

### Foliage treatment test for upland field

Compost mixed soil was filled in 1/3,000 are pots, seeded with southern crabgrass, common lambsquaters, slender amaranth, common purslane, velvetleaf, wheat, corn and soybean. The seeds were covered up with the soil and cultured in a glasshouse kept at 25°C on the average.

Wettable powders containing the object compounds (1) as identified in Table 1, which were formulated following Preparation Example 2, were diluted with water to the prescribed dose of the active ingredient, and uniformly sprayed onto the weeds at 1.0 - 2.0 leaf stage of the southern crabgrass at an application water of 15 liters per are.

After placing the test pots in a grasshouse kept at 25°C on the average for 3 weeks, the herbicidal effect was examined.

Evaluation of the herbicidal effect was conducted in the similar manner to Test Example 1.

The results were as shown in Table 5.

**TABLE 5**

| | | Foliage Treatment | | | | | | | |
|---|---|---|---|---|---|---|---|---|---|
| | | Herbicidal Effect | | | | | Phytotoxicity | | |
| Compound No. | Dose (gai/a) | Southern crabgrass | Common lamb-squatters | Slender amaranth | Common purslane | velvetleaf | wheat | corn | soybean |
| 3 | 2.5 | 5 | 5 | 5 | 5 | 5 | 1 | 1 | 1 |
| | 5 | 5 | 5 | 5 | 5 | 5 | 1 | 1 | 2 |
| 9 | 2.5 | 5 | 5 | 5 | 5 | 4 | 1 | 1 | 0 |
| | 5 | 5 | 5 | 5 | 5 | 5 | 1 | 1 | 1 |
| 11 | 2.5 | 5 | 5 | 5 | 5 | 5 | 1 | 2 | 2 |
| | 5 | 5 | 5 | 5 | 5 | 5 | 1 | 1 | 1 |
| 33 | 2.5 | 4 | 4 | 5 | 5 | 4 | 0 | 0 | 1 |
| | 5 | 4 | 4 | 5 | 5 | 4 | 0 | 0 | 1 |
| 39 | 2.5 | 4 | 5 | 5 | 5 | 4 | 0 | 0 | 1 |
| | 5 | 5 | 5 | 5 | 5 | 5 | 1 | 1 | 1 |
| 46 | 2.5 | 4 | 5 | 5 | 5 | 4 | 0 | 0 | 1 |
| | 5 | 5 | 5 | 5 | 5 | 5 | 1 | 1 | 1 |
| 52 | 2.5 | 5 | 5 | 5 | 5 | 5 | 1 | 1 | 1 |
| | 5 | 5 | 5 | 5 | 5 | 5 | 1 | 1 | 2 |
| 54 | 2.5 | 5 | 5 | 5 | 5 | 5 | 1 | 1 | 2 |
| | 5 | 5 | 5 | 5 | 5 | 5 | 1 | 2 | 2 |
| 82 | 2.5 | 4 | 5 | 5 | 5 | 2 | 0 | 0 | 1 |
| | 5 | | 5 | 5 | 5 | 3 | 0 | 1 | 1 |
| 84 | 2.5 | 4 | 4 | 5 | 4 | 2 | 0 | 0 | 0 |
| | 5 | 4 | 5 | 5 | 5 | 3 | 0 | 1 | 0 |
| 85 | 2.5 | 5 | 5 | 5 | 5 | 5 | 1 | 1 | 2 |
| | 5 | 5 | 5 | 5 | 5 | 5 | 1 | 1 | 2 |
| 87 | 2.5 | 4 | 5 | 5 | 5 | 4 | 0 | 0 | 1 |
| | 5 | 5 | 5 | 5 | 5 | 5 | 1 | 1 | 1 |
| 117 | 2.5 | 4 | 5 | 5 | 5 | 4 | 0 | 0 | 0 |
| | 5 | 5 | 5 | 5 | 5 | 5 | 0 | 1 | 1 |
| 126 | 2.5 | 4 | 5 | 5 | 5 | 3 | 0 | 0 | 1 |
| | 5 | 5 | 5 | 5 | 5 | 4 | 1 | | 1 |
| 151 | 2.5 | 4 | 5 | 5 | 5 | 3 | 0 | 0 | 0 |
| | 5 | 5 | 5 | 5 | 5 | 4 | 0 | 0 | 0 |

## Claims

1. Benzoxazole compounds represented by the formula (1), 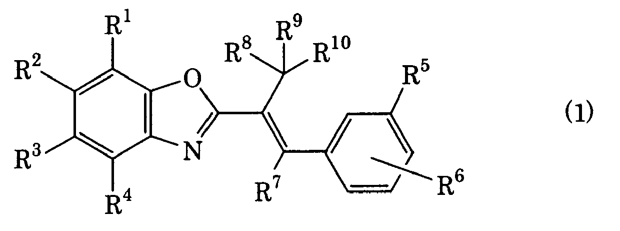 in the formula,
R¹ stands for hydrogen, halogen or C₁₋₄ alkyl,
R² stands for hydrogen, halogen, C₁₋₄ haloalkyl or C₁₋₄ alkyl,
R³ stands for hydrogen, C₁₋₄ haloalkyl, halogen, nitro, C₁₋₄ alkyl, cyano, R¹¹S(O)ₙ, C₁₋₄ haloalkoxy, C₂₋₅ alkanoyl, formyl, C₂₋₆ alkoxycarbonyl or carboxyl, where R¹¹ standing for C₁₋₄ alkyl and n being an integer of 0 - 2,
R⁴ stands for hydrogen, halogen or C₁₋₄ alkyl,
R⁵ stands for C₁₋₄ haloalkyl or cyano,
R⁶ stands for hydrogen, halogen, cyano or C₁₋₄ haloalkyl,
R⁷ stands for hydrogen, halogen or C₁₋₄ alkyl,
R⁸ and R⁹ each independently stands for hydrogen or C₁₋₄ alkyl,
R¹⁰ stands for halogen, cyano or R¹² X, where R¹² standing for C₁₋₁₀ alkyl, C₁₋₆ haloalkyl, C₃₋₆ cycloalkyl, C₂₋₇ alkanoyl, C₂₋₇ haloalkanoyl or formyl; and X standing for oxygen or sulfur.

2. The compounds as set forth in Claim 1, in which
R¹ is hydrogen, halogen or C₁₋₃ alkyl,
R² is hydrogen, halogen, C₁₋₃-haloalkyl or C₁₋₃ alkyl,
R³ is hydrogen, C₁₋₃ haloalkyl, halogen, nitro, C₁₋₄ alkyl, cyano, R¹¹S(O)ₙ, C₁₋₃ haloalkoxy, C₂₋₃ alkanoyl, C₂₋₄ alkoxycarbonyl or carboxyl, wherein R¹¹ is C₁₋₃ alkyl and n is an integer of 0 - 2,
R⁴ is hydrogen, halogen or C₁₋₃ alkyl,
R⁵ is C₁₋₃ haloalkyl or cyano,
R⁶ is hydrogen, halogen, cyano or C₁₋₃ haloalkyl,
R⁷ is hydrogen, halogen or C₁₋₃ alkyl,
R⁸ and R⁹ are hydrogen or C₁₋₃ alkyl, independently of each other,
R¹⁰ is halogen, cyano or R¹² X, wherein R¹² is C₁₋₁₀ alkyl, C₁₋₆ haloalkyl, cyclohexyl, C₂₋₅ alkanoyl, C₂₋₅ haloalkanoyl or formyl and X is oxygen or sulfur.

3. The compounds as set forth in Claim 1, in which
R¹ is hydrogen or halogen,
R² is hydrogen, halogen or C₁₋₃ haloalkyl,
R³ is halogen, cyano, C₁₋₃ haloalkyl, C₁₋₃ haloalkoxy, C₁₋₄ alkyl or C₂₋₃ alkanoyl,
R⁴ is hydrogen,
R⁵ is C₁₋₃ haloalkyl,
R⁶ is hydrogen or halogen,
R⁷ is hydrogen,
R⁸ and R⁹ each is hydrogen,
R¹⁰ is halogen or R¹²X, wherein R¹² is C₁₋₁₀ alkyl, C₁₋₆ haloalkyl, cyclohexyl or formyl, and X is oxygen or sulfur.

4. The compounds as set forth in Claim 1, in which
R¹ is hydrogen, fluorine or chlorine,
R² is hydrogen, fluorine, chlorine or trifluoromethyl,
R³ is fluorine, chlorine, bromine, iodine, trifluoromethyl, cyano, tert-butyl or acetyl,
R⁴ is hydrogen,
R⁵ is trifluoromethyl,
R⁶ is hydrogen, fluorine or chlorine,
R⁷ is hydrogen,
R⁸ and R⁹ each is hydrogen,
R¹⁰ is R¹² X, wherein R¹² is methyl, ethyl, n-propyl, iso-propyl, n-hexyl, n-octyl, n-decyl, 2,2,2-trifluoroethyl, 2,2,3,3-tetrafluoropropyl, 2-chloromethyl, 4-chloro-n-butyl, 6-chloro-n-hexyl or cyclohexyl; and X is oxygen.

5. The compounds as set forth in Claim 1, which are selected from the following groups:
(i) compounds of the formula (1) in which R¹, R², R⁴ and R⁶-R⁹ each stands for hydrogen; R³ stands for halogen; R⁵ stands for C₁₋₄ haloalkyl; and R¹⁰ stands for C₁₋₁₀ alkoxy;
(ii) compounds of the formula (1) in which R¹, R², R⁴ and R⁷ - R⁹ each stands for hydrogen; R³ stands for halogen; R⁵ stands for C₁₋₄ haloalkyl; R⁶ stands for halogen; and R¹⁰ stands for C₁₋₁₀ alkoxy;
(iii) compounds of the formula (1) in which R¹, R², R⁴ and R⁶ - R⁹ each stands for hydrogen, R³ stands for C₁₋₄ haloalkyl; R₅ stands for C₁₋₄ haloalkyl; and R¹⁰ stands for C₁₋₁₀ alkoxy;
(iv) compounds of the formula (1) in which R¹, R², R⁴ and R⁶-R⁹ each stands for hydrogen; R³ stands for C₁₋₄ haloalkyl, R⁵ stands for C₁₋₄ haloalkyl; and R¹⁰ stands for C₁₋₆ haloalkoxy;
(v) compounds of the formula (1) in which R^{2,} R⁴ and R⁶ - R⁹ each stands for hydrogen; R¹ and R³ each stands for halogen; R⁵ stands for C₁₋₄ haloalkyl; and R¹⁰ stands for C₁₋₆ alkoxy;
(vi) compounds of the formula (1) in which R¹, R⁴ and R⁶ - R⁹ each stands for hydrogen; R² and R³ each stands for halogen; R⁵ stands for C₁₋₄ haloalkyl; and R¹⁰ stands for C₁₋₁₀ alkoxy;
(vii) compounds of the formula (1) in which R¹, R², R⁴ and R⁶ - R⁹ each stands for hydrogen; R³ stands for C₁₋₄ alkyl; R⁵ stands for C₁₋₄haloalkyl; and R¹⁰ stands for C₁₋₁₀ alkoxy;
(viii) compounds of the formula (1) in which R¹, R⁴ and R⁶ - R⁹ each stands for hydrogen; R² stands for halogen; R³ stands for cyano; R⁵ stands for C₁₋₄ haloalkyl; and R¹⁰ stands for C₁₋₁₀ alkoxy; and
(ix) compounds of the formula (1) in which R¹, R⁴ and R⁶ - R⁹ each stands for hydrogen; R² stands for C₁₋₄ haloalkyl; R³ stands for halogen; R⁵ stands for C₁₋₄ haloalkyl; and R¹⁰ stands for C₁₋₁₀ alkoxy.

6. Methods for preparing compounds of the formula (1), which comprise: reacting a compound of the following formula (2): 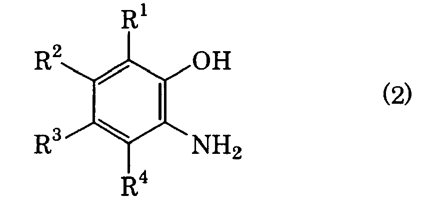 in which R¹-R⁴ have the same significations as given in Claim 1, with a compound represented by the following formula (3): 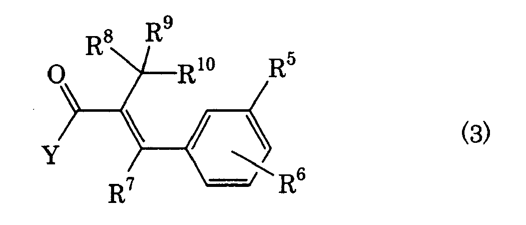 in which R⁵-R¹⁰ have the same significations as given in Claim 1 and Y stands for halogen, in the presence of a base or acid catalyst; or a method for preparing the compounds of the formula (1) in which R¹⁰ stands for halogen, i.e., compounds of the following formula (5): 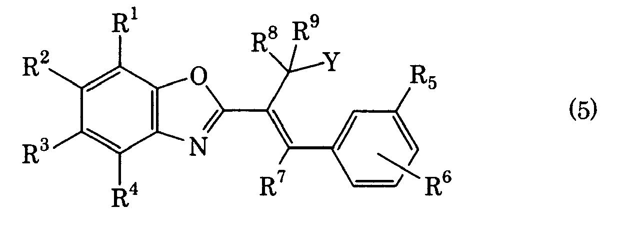 in which R¹-R⁹ have the same definitions as given in Claim 1; and Y stands for halogen,
which comprises reacting a compound of the following formula (4): 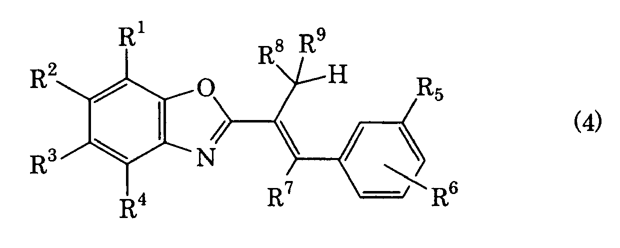 in which R¹-R⁹ have the same definitions as given in Claim 1, with a halogenating agent; or a method for preparing the compounds (1) in which R¹⁰ stands for R¹² X, i.e., compounds of the following formula (7): 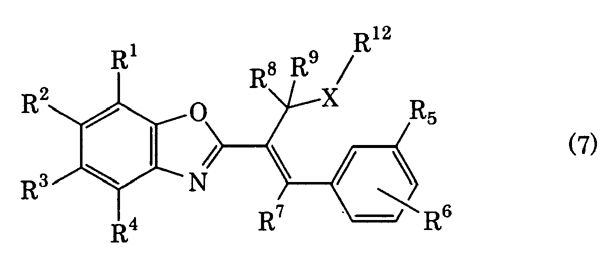 in which R¹-R⁹, R¹² and X have the same definitions as given in Claim 1, which comprises reacting a compound of the following formula (5): 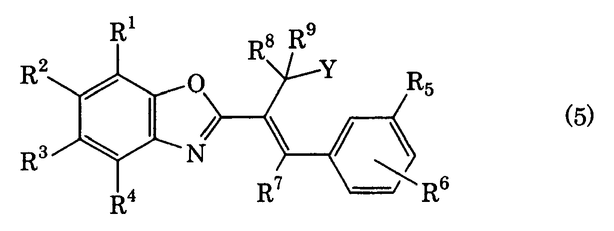 in which R¹-R⁹ have the same definitions as given in Claim 1, and Y stands for halogen, with a compound represented by the following formula (6):
R¹² -X-M (6)
in which R¹² and X have the same definitions as given in Claim 1 and M stands for hydrogen or alkali metal,
in the presence of a base catalyst.

7. Herbicides which are **characterized by** comprising compounds of the formula (1) as given in Claim 1 as active ingredient.

8. Herbicidal compositions which are **characterized by** comprising compounds of the formula (1) as given in Claim 1 and agriculturally suitable adjuvants.

9. A method for controlling weeds which is **characterized by** applying an effective amount of a compound of the formula (1) as given in Claim 1 to weeds or their growing sites.

10. Use of compounds of the formula (1) as given in Claim 1 as herbicide.
